(19) Europäisches Patentamt
European Patent Office
Office européen des brevets

(11) Numéro de publication : **0 100 254**
**B1**

(12) # FASCICULE DE BREVET EUROPÉEN

(45) Date de publication du fascicule du brevet : **20.09.89**

(51) Int. Cl.⁴ : **C 12 N 15/00**, C 12 N 9/42, C 12 N 1/20// C12R1/19

(21) Numéro de dépôt : **83401375.7**

(22) Date de dépôt : **04.07.83**

(54) **Micro-organismes, notamment-E. coli, transformés par une séquence d'ADN orginaire de C. thermocellum, compositions à activité cellulolytique issues de ces micro-organismes et procédé pour les obtenir.**

(30) Priorité : **02.07.82 FR 8211698**

(43) Date de publication de la demande :
**08.02.84 Bulletin 84/06**

(45) Mention de la délivrance du brevet :
**20.09.89 Bulletin 89/38**

(84) Etats contractants désignés :
**AT BE CH DE FR GB IT LI LU NL SE**

(56) Documents cités :
**FEMS MICROBIOLOGY LETTERS, vol. 16, 1983, pages 137-141, Elsevier Biomedical Press, P. CORNET: "Cloning and expression in Escherichia coli of Clostridium thermocellum genes coding for amino acid synthesis and cellulose hydrolysis"**
**DEVELOPMENTS IN INDUSTRIAL MICROBIOLOGY, vol. 22, 1981, pages 87-95; R.F. GOMEZ: "Developmnt of genetic principles in Clostridium thermocellum"**
**GENE, vol. 17, 1982, pages 139-145, Elsevier Biomedical Press, D.J. WHITTLE et al. "Molecular cloning of a Cellulomonas fimi cellulase gene in Escherichia coli"**
**CHEMICAL ABSTRACTS, vol. 97, no. 1, juillet 1982, page 457, no. 4552g, Columbus, Ohio, USA, A.P. JAMES et al.: "Applications of recombinant DNA technology for the utilization of cellulose by yeast" Proceeding Bioenergy RRD 135-39, 1981**

(73) Titulaire : **INSTITUT PASTEUR**
**28, rue du Docteur Roux**
**F-75715 Paris Cedex 15 (FR)**

(72) Inventeur : **Cornet, Philippe**
**26, Avenue Guy de Maupassant**
**F-78400 Chatou (FR)**
Inventeur : **Millet, Jacqueline**
**114, rue de Grenelle**
**F-75007 Paris (FR)**
Inventeur : **Aubert, Jean-Paul**
**5, rue Gazan**
**F-75014 Paris (FR)**

(74) Mandataire : **Gutmann, Ernest et al**
**S.C. Ernest Gutmann - Yves Plasseraud 67, boulevard Haussmann**
**F-75008 Paris (FR)**

**EP 0 100 254 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

L'invention concerne des moyens nouveaux, plus particulièrement des micro-organismes et des procédés nouveaux de production d'enzymes cellulolytiques capables de réaliser la bioconversion des celluloses, hémicelluloses et de leurs dérivés, plus généralement de matières fermentescibles du type oligosaccharides et monosaccharides, lesquelles peuvent à leur tour être transformées par fermentation notamment en éthanol, acide acétique et acide lactique. Elle concerne encore des compositions ou sources de telles enzymes pouvant être utilisées telles quelles dans des unités industrielles de transformation de matières contenant des proportions substantielles de cellulose ou dérivés de cellulose. A titre d'exemple de matières ainsi transformables, on peut citer les matières de rejet municipales (ordures) ou industrielles, ou encore de matières végétales telles que la paille.

La bioconversion de matériaux cellulosiques connaît à ce jour un regain d'intérêt ; comme l'attestent les publications par exemple de Zeikus, J.G. (1980) Ann. Rev. Microbiol. 34, 423-464 ou de Bisaria, V.S. et Ghose, T.K. (1981) Enzyme Microbiol. Technol. 3, 90-104.

A.P. James et al, Proc. Bioenergy R & D Semin. 1981, 3$^{rd}$, 135-9, exposent un projet, dont l'objet était de créer génétiquement une levure capable de fermenter directement la cellulose en éthanol. Les auteurs décrivent la fabrication d'une banque de fragments d'ADN obtenus à partir des génomes de Trichoderma reesei et Schizophyllum commune et l'isolement d'un clone de phage $\lambda$ recombinant, témoignant, dans un hôte E. coli, d'une « apparente activité cellulosique », détectée par électrophorèse sur un gel d'agarose.

De même D.J. Whittle et al, Gene, 17 (1982) 139-145 décrivent le clonage moléculaire dans E. coli d'un gène issu de Cellulomonas fini et codant pour une cellulase.

Une attention particulière a également été portée au micro-organisme connu sous la désignation Clostridium thermocellum, lequel est apte à produire des quantités importantes d'une enzyme cellulolytique thermostable hautement active, par exemple en des proportions qui peuvent atteindre de l'ordre de 3 mg d'enzyme par litre de culture, après 50 heures d'incubation.

L'utilisation de C. thermocellum ne va cependant pas sans difficultés, dans la mesure où il s'agit d'un micro-organisme anaérobie strict, thermophile. Ces caractéristiques en font un micro-organisme dont la culture dans des conditions industrielles est relativement malaisée, le micro-organisme n'étant apte à produire les enzymes cellulolytiques qu'à la condition d'être cultivé à température élevée, notamment à une température de l'ordre de 60 °C, rigoureusement à l'abri de l'oxygène.

L'invention a pour but de fournir des moyens nouveaux de production d'enzymes cellulolytiques, plus faciles à mettre en œuvre, plus particulièrement aptes à produire des quantités importantes d'enzyme dans un domaine de température plus accessible aux techniques usuelles de fermentation, sans qu'il soit nécessaire par ailleurs d'opérer la mise en œuvre de ces moyens en l'absence d'oxygène.

L'invention découle de la découverte que des séquences suffisamment courtes de l'ADN de C. thermocellum et contenant une séquence de nucléotides codant pour une enzyme cellulolytique pouvait être utilisée pur la transformation de micro-organismes différents ; ceux-ci devenant alors capables de fabriquer à leur tour des quantités suffisamment importantes d'une enzyme cellulolytique, pour que l'on puisse envisager leur substitution à C. thermocellum, en tant que source d'une enzyme cellulolytique du genre en question. Alors que C. thermocellum n'est capable de produire ces enzymes que lorsqu'il est incubé en milieu strictement désoxygéné et à une température élevée, on a constaté que d'autres micro-organismes, notamment des bactéries aérobies, étaient capables de la produire avec des rendements satisfaisants dans leurs conditions de culture normales et à des températures beaucoup plus faibles.

A titre d'exemple de ces micro-organismes, on mentionnera surtout Escherichia coli. Ce dernier type de micro-organisme se révèle particulièrement intéressant en raison de la rapidité du développement de ses cultures. Cette rapidité, combinée avec les quantités d'enzymes que ces micro-organismes sont capables de produire, permet d'envisager la production de quantités d'enzyme par unités de temps, du même ordre de grandeur que celles qui peuvent être obtenues, dans les conditions beaucoup plus difficiles qui ont été évoquées plus haut, à partir de C. thermocellum.

L'invention concerne par conséquent des micro-organismes distincts de C. thermocellum, aérobies stricts ou facultatifs, transformés par un vecteur contenant un insérat issu du patrimoine génétique de C. thermocellum et contenant une séquence d'ADN codant pour une enzyme cellulolytique, cet insérat étant placé dans ledit vecteur, dans des conditions permettant l'expression dans ce micro-organisme distinct de C. thermocellum de la séquence codant pour l'enzyme cellulolytique.

Cette séquence est de préférence de longueur suffisamment réduite, de préférence n'excédant pas de l'ordre de 40 kilobases (kb) pour permettre au micro-organisme hôte d'en produire une quantité suffisante, telle qu'appréciée notamment par le degré d'activité enzymatique des extraits cellulaires obtenus à partir desdites cultures, vis-à-vis de substrats déterminés. De préférence, la susdite séquence a une longueur qui est inférieure à 3 kb.

Plus particulièrement l'invention concerne des micro-organismes transformés, notamment des E. coli transformés par une séquence d'ADN issue de C. thermocellum et reconnaissables par la capacité de l'enzyme d'induire une diminution de la viscosité de solutions initialement visqueuses de carboxyméthyl-cellulose dissoute dans un tampon approprié. Les micro-organismes ainsi transformés seront quelquefois dits « $CMC^+$ », dans la suite de cette demande.

Les micro-organismes transformés par les séquences d'ADN selon l'invention peuvent encore être détectés en ayant recours à une modification de la technique de Teather, R.M. et Wood, P.J. (1982, « Use of Congo redpolysaccharide interactions in enumeration and characterization of cellulolytic bacteria from the bovine rumen ». Appl. Environ. Microbiol. 43 : 777-780). Cette méthode repose sur la détection de l'activité CMCase sur plaques. Par exemple, lorsque la technique est appliquée à des clones de E. coli, ceux-ci sont mis en culture pendant une nuit sur un milieu solide LB décrit par Miller, J.H. (1972, « In Experiments in molecular genetics ». Cold Spring Harbor Laboratory, Cold Spring Harbor, New York) à 37 °C. Les plaques sont ensuite recouvertes avec 5 ml du tampon PC (dont la composition est indiquée ci-après) contenant en outre de l'agar à 0,7 % et de la carboxyméthylcellulose (CMC) à 0,5 %. Après 3 heures d'incubation à 60 °C, les plaques sont recouvertes d'une solution aqueuse de rouge Congo à 1 %, pendant 1 minute, à la température ambiante. La solution de rouge Congo est ensuite enlevée et les plaques sont lavées avec une solution de NaCl 1M. Les clones actifs, capables de synthétiser la CMCase apparaissent alors avec un halo jaune se détachant sur un fond rouge.

L'invention concerne en particulier des E. coli transformés, dont les extraits bruts obtenus après culture des cellules, rupture et séparation des parois cellulaires, conduisent, lorsqu'ils sont mis en contact avec une solution de 1,5 % (poids/volume) de carboxyméthylcellulose dans un tampon PC (50 mM $K_2HPO_4$ ; acide citrique 12,5 mM ; pH 6,3) à raison d'une quantité d'extrait brut contenant 65 µg du même tampon, par 1 ml de la susdite solution, à une réduction de moitié de la viscosité initiale après 30 minutes et de préférence 10 minutes d'incubation du mélange formé à 60 °C.

On peut avoir recours pour mesurer la diminution de viscosité à toute technique en soi connue, par exemple à la mesure des variations du temps de passage du mélange avant et après incubation à travers des pipettes calibrées du type de celles utilisées couramment en viscosimétrie. Ces mesures peuvent naturellement être effectuées, en ayant recours à tout autre dispositif classique de viscosimétrie.

De préférence ces E. coli transformés sont également ceux dont les mêmes extraits bruts conduisent, après contact avec la carboxyméthylcellulose, dans les mêmes proportions que dans le test précédent, à une activité CMCase exprimée en % d'équivalents de glucose libéré d'au moins 0,3 %, de préférence au moins 1 %, après incubation du mélange à 60 °C pendant 1 heure.

Des micro-organismes transformés préférés selon l'invention sont encore ceux qui, outre les caractéristiques quantitatives déjà indiquées, présentent l'une ou l'autre ou encore l'une et l'autre des caractéristiques supplémentaires qui suivent.

La première de ces caractéristiques supplémentaire consiste en la capacité des extraits desdits micro-organismes d'hydrolyser la trinitrophényl-carboxyméthylcellulose (TNP-CMC ou TCMC). Ces micro-organismes seront ci-après dits « $TCMT^+$ ». Les micro-organismes préférés sont ceux dont les extraits bruts sus-indiqués présentent, lorsqu'ils sont utilisés à raison d'une quantité correspondant à 65 µg de protéines, une activité TNP-CMCase d'au moins 0,05 unité d'absorbance par heure, après incubation du mélange à 60 °C.

Il est entendu que, dans ce qui précède, les mesures de variations d'absorbance d'une radiation à une longueur d'onde de 350 nm, s'expriment par rapport à l'action du mélange sur le substrat chromogène constitué par la TNP-CMC, préparée selon la méthode de Huang et Tang (Anal. Biochem. (1976), 73, 369-377) et par rapport à l'absorption mesurée sur un mélange des constituants évoqués plus haut, après incubation dans les mêmes conditions, cependant en l'absence de l'extrait cellulaire.

La seconde des caractéristiques sus-visées desdits micro-organismes consiste en la capacité de l'enzyme cellulolytique contenue dans lesdits extraits d'être reconnue immunologiquement, notamment par exemple dans des tests d'immunodiffusion du type Ouchterlony, par des anticorps préalablement préparés contre l'endo-β-1, 4-glucanase extracellulaire de C. thermocellum, obtenue et purifiée selon la technique décrite par J. Petre, R. Longin et J. Millet dans Biochimie, 1981, 63, 629-639.

Les micro-organismes modifiés sont obtenus à partir de micro-organismes transformables par un vecteur déterminé, lui-même dérivé soit d'un plasmide, soit d'un phage, soit encore d'un cosmide, ce vecteur ayant cependant au préalable été lui-même modifié par un insérat issu de C. thermocellum et contenant une séquence d'ADN codant pour une enzyme cellulolytique.

Il va de soi que l'expression « insérat issu de C. thermocellum » signifie soit une séquence d'ADN provenant directement du patrimoine génétique de C. thermocellum, en particulier d'un gène de ce micro-organisme, soit une séquence d'ADN susceptible de coder pour la même enzyme cellulolytique, notamment des séquences résultant du clonage soit du précédent gène, soit d'un cADN correspondant.

De préférence, et plus particulièrement lorsque le micro-organisme transformé est constitué par E. coli, on choisit ceux des insérats contenant une séquence codant pour une enzyme cellulolytique qui permette, après transformation et culture de ces micro-organismes, l'obtention d'une production par le micro-organisme de l'enzyme cellulolytique en quantité et activité suffisantes pour satisfaire au moins aux conditions minimales définies plus haut.

Suivant un mode de réalisation préféré de l'invention, l'insérat consiste en une séquence issue de C. thermocellum contenant non seulement le gène ou ADN correspondant codant pour l'enzyme cellulolytique, mais aussi les séquences d'ADN contrôlant l'expression du gène dans C. thermocellum. En particulier l'insérat contient le promoteur sous le contrôle duquel est réalisée l'expression du gène dans C. thermocellum. Il va de soi que cette condition n'est pas essentielle et que le gène codant pour l'enzyme cellulolytique peut être remplacé par une partie seulement de ce gène ou par une séquence d'ADN

équivalente, dans la mesure où il code pour un polypeptide comportant des propriétés cellulolytiques. L'invention s'étend naturellement également aux micro-organismes dans lesquels le gène (ou la partie de gène ou séquence d'ADN équivalente) aurait au préalable été placé sous le contrôle de tout promoteur distinct reconnu par les « machineries » assurant la transcription et la traduction de protéines déterminées dans le micro-organisme hôte, notamment un promoteur endogène sous le contrôle duquel se trouvent placés des gènes naturels et susceptibles d'être exprimés dans ce micro-organisme.

L'invention fournit également un procédé permettant la sélection de tels insérats, ce procédé consistant à fragmenter l'ADN de C. thermocellum par une enzyme de restriction permettant la production d'un nombre de fragments suffisant, par exemple des fragments Sau 3 AI, l'insertion de ces fragments dans un vecteur, la transformation de cultures du micro-organisme avec les différents vecteurs modifiés obtenus et la sélection de celles des cultures de cellules productrices de l'enzyme cellulolytique dans les conditions sus-indiquées. Dans un mode de mise en œuvre préféré de ce procédé de sélection, on a recours à des vecteurs du type cosmide, lesquels permettent l'empaquetage relativement sélectif de fragments comportant notamment de 25 à 50 kb, d'où la possibilité d'obtenir des clones en nombre relativement limité, de sorte que les opérations de sélection s'en trouvent facilitées.

L'invention repose en effet sur la capacité de reconnaissance par des hôtes étrangers, notamment par E. coli, des séquences d'ADN et ARN qui contrôlent l'expression des gènes de C. thermocellum, en tous cas d'un certain nombre de gènes susceptibles d'exprimer une enzyme cellulolytique.

La capacité des micro-organismes transformés obtenus de synthétiser des enzymes cellulolytiques normalement produites par C. thermocellum est aussi d'autant plus remarquable que le micro-organisme hôte, et plus particulièrement E. coli ne dispose vraisemblablement pas de la « machinerie cellulaire » permettant l'excrétion de l'enzyme ainsi synthétisée dans le milieu de culture et, par conséquent, la maturation qui très vraisemblablement l'accompagne, dans les cultures de C. thermocellum.

Partant d'un clone actif, il est naturellement possible de réisoler l'insérat, d'en faire une analyse de séquences et par refragmentation, d'en isoler des fragments plus petits, de les réinsérer dans un vecteur approprié, tel qu'un plasmide ou un phage, pour déterminer de nouveau ceux des plasmides ou phages ainsi transformés qui, à leur tour, peuvent à nouveau transformer un micro-organisme du même type et finalement de sélectionner ceux des micro-organismes alors rendus capables de synthétiser l'enzyme cellulolytique.

En particulier, cette fragmentation peut être réalisée par des délétions mettant en jeu des enzymes de restriction, dans la mesure où ces délétions n'entraînent pas la perte de la capacité du micro-organisme transformé, avec les insérats ainsi modifiés, d'exprimer une enzyme cellulolytique. Partant d'un plasmide contenant un insérat susceptible d'être exprimé dans un micro-organisme donné, on peut linéariser au niveau d'un site de restriction, manifestement extérieur à la séquence d'ADN intervenant dans l'expression (notamment un site de restriction du vecteur extérieur à l'insérat), émonder de façon contrôlée le plasmide linéarisé avec une enzyme exonucléolytique, telle que l'enzyme Bal31, recirculariser le plasmide au moyen d'un ADN ligase, transformer un micro-organisme approprié, initialement transformable par le même vecteur, et détecter, parmi les produits éventuels d'expression du micro-organisme ainsi transformé, l'éventuelle présence d'une enzyme ou polypeptide cellulolytique, en utilisant notamment l'un des tests mentionnés plus haut, le cycle d'opérations qui vient d'être défini étant répété jusqu'à la disparition de la détection de l'enzyme ou polypeptide cellulolytique antérieurement exprimé par le micro-organisme.

Il va de soi qu'une telle opération n'est sérieusement envisageable que dans la mesure où l'on détiendra déjà un fragment d'ADN issu de C. thermocellum ayant une taille suffisamment réduite, pour que le cycle d'opérations qui vient d'être sommairement indiqué n'ait pas besoin d'être répété un trop grand nombre de fois. Ces opérations, éventuellement complétées par des analyses de séquences appropriées, sont réalisables par l'homme du métier avec les connaissances dont il dispose à ce jour, que ce soit pour isoler une séquence d'ADN correspondant à l'ensemble gène-éléments de régulation dans C. thermocellum ou pour isoler le gène séparé de son promoteur naturel, dans la mesure où il est destiné à être mis (en phase) sous le contrôle d'un promoteur distinct.

L'invention concerne enfin plus particulièrement les compositions nouvelles présentant une activité cellulolytique, plus particulièrement du type endo-β-1,4-glucanase, directement applicables au traitement de matières cellulosiques, notamment à une température de l'ordre de 50 à 70 °C, de préférence de l'ordre de 60 °C, ces compositions étant constituées :

soit par la masse des micro-organismes transformés, plus particulièrement de E. coli dans les conditions indiquées plus haut, après séparation du milieu de culture et rupture des parois, soit mécaniquement, soit par lyse des parois cellulaires, la composition obtenue pouvant être séchée et/ou utilisée telle quelle,

soit par une composition davantage concentrée ou purifiée en principes actifs, obtenue à partir de la préparation brute ci-dessus. Dans ce dernier cas, la composition selon l'invention est formée par les extraits hydrosolubles obtenus après séparation des parois bactériennes et autres résidus purifiés, par exemple jusqu'à atteindre une activité TNP-CMCase de 0,5 unité d'absorbance par heure d'incubation à 60 °C.

La préparation des compositions brutes peut être réalisée par toute méthode en soi connue. En particulier, on peut avoir recours à des procédés mettant éventuellement en jeu une ou des substances

chimiques susceptibles de l'accélérer, sans pour autant altérer l'activité enzymatique des préparations en cause, lorsqu'un traitement de simple dessication de la culture ne suffirait pas à causer les ruptures des parois.

On peut d'autant plus facilement faciliter cette autolyse par une certaine élévation de température que l'enzyme cellulolytique recherchée est thermostable. L'élévation de température présente même l'avantage d'inactiver si besoin la majeure partie des autres enzymes contenues dans les micro-organismes ainsi traités.

Le contenu des cellules ainsi rompues peut être récupéré de toute façon en soi connue, notamment après séparation des parois rompues. Aux extraits obtenus peuvent être réunies les solutions de lavage des résidus insolubles. On peut avoir recours pour effectuer ce lavage à toute solution tamponnée compatible avec la préservation de l'activité enzymatique recherchée.

La solution obtenue peut ensuite être soumise à des traitements de purification supplémentaire, en ayant recours à des techniques en soi bien connues, par exemple comme celles décrites dans « Encyclopedia of Chemical Technology » de Kirk Othmer, vol. 8, 2e édition.

L'invention concerne enfin le procédé de traitement de matières contenant des substances cellulosiques, en vue de produire des substrats du type mono- ou oligo-saccharides eux-mêmes fermentescibles en produits utiles, tels que l'éthanol, l'acide acétique et l'acide lactique, caractérisé par la mise en contact de ces matières avec les compositions enzymatiquement actives, selon l'invention, à une température comprise entre 50 et 70 °C, notamment de l'ordre de 60 °C, pendant le temps nécessaire à la réalisation de la lyse contrôlée desdites matières cellulosiques, plus particulièrement de leurs liaisons β-1,4 glycosyle.

Des caractéristiques supplémentaires de l'invention apparaîtront encore au cours de la description qui suit, à titre non limitatif, d'exemples d'obtention de micro-organismes transformés enzymatiquement actifs, et de leurs niveaux d'activité. On se réfèrera à cet égard aux dessins, dans lesquels :

la fig. 1 contient des courbes représentatives des activités enzymatiques, obtenues à partir d'extraits de micro-organismes transformés, dans des tests d'activité différents ;

les fig. 2a et 2b sont des cartes de restriction de plasmides contenant des séquences codant pour des enzymes cellulolytiques de C. thermocellum ;

les fig. 3a et 3b représentent schématiquement les étapes de l'insertion de séquences d'ADN excisées des plasmides précédents dans pBR322 ;

la fig. 4 représente des cartes de restriction de fragments d'ADN issus du génome de C. thermocellum et contenant les gènes codant pour des enzymes cellulolytiques ;

la fig. 5 représente des cartes de restriction de parties des plasmides dérivés de pBR322 (fig. 3a et fig. 3b) ou des plasmides également dérivés de pBR322 et contenant des insérats issus des séquences d'ADN du génome de C. thermocellum, après hybridation avec les insérats des plasmides modifiés des fig. 3a et 3b et clonage de ces séquences, également dans pBR322.

On a utilisé, au titre des micro-organismes transformables, E. coli 4282 (leu B6, trp E 38, met E 70 rec A, sup. E) (déposé au E. coli Genetic Stock Center à la Yale University et décrit par Berg et Curtiss 1967, Genetics 56, 503 et déposé également le 2 juillet 1982 à la C.N.C.M. sous le nº I-197) et E. coli HB101 (pro leu thi hsdR rec A) (déposé à la C.N.C.M. sous le nº I-196). Le cosmide utilisé a été pHC79, décrit par Hohn B. et Collins J. (1980) Gene, 11, 291-298 et commercialisé par Boehringer Mannheim.

Obtention de l'ADN de C. thermocellum

De l'ADN total de C. thermocellum, obtenu à partir d'une culture de la souche NCIB 10682 dans le milieu complet décrit par Petre J. et coll. (1981) Biochimie 63, 629-639 a été isolé par la méthode décrite par Marmur J. (1961) J. Mol. Biol. 3,208-218, cependant modifiée par un traitement supplémentaire avec de la protéinase K (1 mg/ml). L'ADN de plasmide tel qu'obtenu à partir des cultures de E. coli 4 282 a été purifié selon la méthode de Humphreys F. et coll. (1975) Biochim. Biophys. Acta 383, 457-463. On a également eu recours aux techniques décrites par Birnboim, H.C. et Doly, J. (1979) Nucl. Acids. Res., 7,1513-1523, pour les extractions rapides d'ADN à partir de cultures de 1 ml.

Construction d'une banque de gènes de C. thermocellum

Des échantillons contenant 5 μg d'ADN de C. thermocellum ont été digérés en présence de 0,5 unité de l'enzyme Sau3AI (Biolabs) pendant des durées de 5, 10 et 20 minutes. Après digestion, les échantillons ont été réunis. 5 μg d'ADN du mélange ont été ajouté à 8 μg de pHC 79, préalablement digérés par l'enzyme de restriction BamHI (Biolabs), au sein d'un volume total de 30 μl. Après ligation en présence de 1 unité de $T_4$ DNA-ligase (Boehringer) pendant 16 heures à 10 °C, l'ADN a été purifié par extraction au phénol et précipitation par l'éthanol, puis remis en suspension dans 30 μl d'un tampon d'empaquetage tel que décrit par Hohn B. et Collins J. déjà mentionnés plus haut. 15 μl de cette solution ont été alors utilisés pour réaliser l'empaquetage in vitro dans des têtes de phages λ, également selon la méthode de Hohn et Collins. Les phages obtenus ont été utilisés pour infecter des cultures de E. coli 4 282. Mille cinq cents clones ampicilline-résistants ($Amp^r$) ont été isolés. Parmi ces derniers 1 200 se sont également révélés $Tet^s$. Ils constituent la susdite banque de gènes de C. thermocellum.

Une partie des 1 200 clones Amp$^r$ Tet$^s$ ont ensuite été analysés du point de vue de leur contenu en ADN plasmidique. Les tailles des insérats se sont révélées varier de 25 à 40 kb. Les cosmides recombinants ainsi obtenus ont été numérotés de 1 à 1 200 (antérieurement de pCT1 à pCT1200).

Les clones de E. coli 4 282 ont été mis en culture pendant la nuit dans le milieu connu sous le nom de « bouillon de Luria » (Miller, J.H., Experiments in Molecular Genetics ; Cold Spring Harbour Laboratory, 1972, page 433) contenant 50 µg/ml d'ampicilline. Les cellules sont reprises dans le tampon PC jusqu'à obtenir une valeur d'absorption de 15 à 570 nm.

Les cellules sont alors rompues mécaniquement par sonication. La solution obtenue, après séparation des fractions insolubles, a été utilisée telle quelle dans les essais pour mesurer la diminution de la viscosité d'une solution de CMC et pour mesurer la libération de groupes réducteurs dans cette même solution.

Pour ceux des clones qui ont été retenus comme capables de produire une enzyme cellulolytique, on a également réalisé des extraits bruts à partir de suspensions de cellules ayant une densité optique de 75 à 570 nm, et l'on a soumis lesdits extraits bruts obtenus aux étapes de purification supplémentaires qui suivent :

chauffage de la solution obtenue à 60 °C pendant 5 minutes,

précipitation des protéines par addition de sulfate d'ammonium jusqu'à atteindre une concentration finale de 90 % de saturation,

reprise du précipité dans le tampon PC,

dialyse de la précédente solution contre le tampon PC, jusqu'à élimination du sulfate d'ammonium avec ajustement de concentration de la solution enzymatique par l'Aquacid III® (Calbiochem). La concentration finale est de 1/25 par rapport au volume initial de l'extrait brut.

Détection des clones producteurs d'une activité cellulolytique

Les extraits bruts des 1 200 clones de la banque ont été réunis 10 par 10 et les extraits bruts obtenus testés pour leur activité CMCase par la méthode viscosimétrique, en raison de l'extrême sensibilité de cette dernière. Deux des extraits bruts mélangés permirent d'obtenir des résultats positifs. Le test fut alors répété sur les extraits bruts obtenus à partir des clones isolés correspondants. Deux clones porteurs des cosmides pCT182 et pCT262, ensuite redésignés par pCT1 et pCT2, avaient acquis la capacité d'induire la dépolymérisation de la CMC. Ce sont les nouvelles désignations de pCT1 et pCT2 qui sont utilisées pour ces clones dans ce qui suit.

La méthode de viscosimétrie utilisée pour apprécier l'effet de dépolymérisation induit par les extraits bruts obtenus à partir des clones pCT1 et pCT2, a été décrite par Almin K.E. et Coll. (1975) Eur. J. Biochem. 51, 207-211. Le principe de la méthode a été rappelé plus haut en rapport avec la définition des micro-organismes préférés de l'invention (E. coli). La variation de viscosité a été appréciée par la mesure du temps de passage d'un échantillon du mélange réactionnel de 0,2 ml à travers une pipette Kimax 51® n° 37 022 de 0,2 ml, à température ambiante. Le mélange était constitué par 1 ml de CMC à 1,5 % dans le tampon PC et 50 µl d'extrait brut.

Les courbes 1 et 4 de la fig. 1 sont représentatives des variations de ce temps de passage, en secondes, en fonction de durées d'incubation croissantes telles qu'obtenues à partir des mélanges de CMC et, respectivement, des extraits de E. coli (pCT1) et E. coli (pCT2). Les extraits bruts obtenus à partir des deux clones sus-indiqués ont également été testés du point de vue de leur activité CMCase et TNP-CMCase dans des conditions qui ont également été indiquées plus haut, en rapport avec les catégories d'E. coli transformées préférées selon l'invention. En ce qui concerne l'activité CMCase, on remarquera qu'elle a été appréciée par la mesure de la quantité de sucres réducteurs libérés à partir de la CMC, par la méthode de Somogyi-Nelson (1944) J. Biol. Chem. 153, 375-380.

Les courbes 2 et 3 sont respectivement représentatives des variations des activités CMCase (en % d'équivalents glucose libérés sur l'axe des ordonnées) et TNP-CMCase (en unités d'absorbance sur un autre axe des ordonnées) des extraits bruts obtenus à partir des E. coli transformés par pCT1. Les courbes 5 et 6 sont représentatives des activités CMCase et TNP-CMCase appréciées dans les mêmes conditions que celles des E. coli transformés par pCT2.

On remarque à l'examen de la fig. 1 que les extraits, tant d'E. coli 4282 (pCT1) que d'E. coli 4282 (pCT2) induisent d'une décroissance rapide de viscosité des solutions de carboxyméthylcellulose avec, dans chaque cas, un accroissement progressif de la quantité de sucres réducteurs libérés en fonction du temps. Par contre, seul E. coli 4282 (pCT1) s'est également révélé posséder une activité TNP-CMCase significative. Des essais réalisés avec les extraits bruts obtenus à partir de E. coli 4.282 transformés par le vecteur pHC 79, utilisés à titre de témoins, se sont révélés ne posséder aucune activité détectable CMCase ou TNP-CMCase.

E. coli 4282 (pCT1) et E. coli 4282 (pCT2) sont déposés à la C.N.C.M. sous les n$^{os}$ I-228 et I-229, respectivement, le 4 juillet 1983.

Dans ce qui suit, le gène codant pour l'enzyme cellulolytique contenue dans pCT1 sera dénommé celA et celui contenu dans pCT2 sera appelé ce1B.

Les plasmides pCT101 et pCT203 dont les cartes de restriction sont indiqués dans les fig. 2a et 2b ont été obtenus au terme de la délétion d'un fragment EcoRI initialement contenu dans les insérats de pCT1

et pCT2, respectivement. Ces délétions (de plus de 9/10 des insérats initiaux) n'ont pas entraîné la perte de la capacité des plasmides recircularisés (au moyen d'un ADN-ligase) de rendre des cellules de E. coli capables d'exprimer des enzymes cellulolytiques apparemment identiques à celles qui étaient exprimées par pCT1 et pCT2 respectivement. En d'autres termes, les deux plasmides pCT101 et pCT203 étaient toujours aptes à conférer le phénotype $CMC^+$ à E. coli. Les délétions se sont donc manifestées par une réduction des tailles des plasmides concernés, de 35 kb à 12,5 kb en ce qui concerne pCT1 et de 40 kb à 10,3 kb en ce qui concerne pCT2. Il est à remarquer que dans les figures 2a et 2b les parties en trait plein sont originaires du cosmide initial pHC79 et les parties en trait fin de la séquence d'ADN de C. thermocellum. Les fragments délimités par des sites HindIII peuvent encore être délétés sans perte par les plasmides recircularisés, qui peuvent être obtenus après recircularisation, de conférer le caractère $CMC^+$ à des cellules de E. coli. L'excision des fragments SalI-HindIII et BamHI-HindIII (dont les positions respectives dans les plasmides (pCT101 et pCT203 sont schématisées par des arcs en traits interrompus dans les fig. 2a et 2b) et leur insertion dans pBR322, selon les schémas des figures 3a et 3b, ont conduit aux plasmides pCT103 et pCT207 (de 6,8 et 6,7 kb, respectivement), qui sont toujours encore aptes à conférer le phénotype $CMC^+$ à E. coli et à rendre ce dernier type de micro-organisme capable de synthétiser des quantités importantes des enzymes cellulolytiques correspondantes.

Les susdites séquences SalI-HindIII et BamHI-HindIII ont également été utilisées pour détecter après hydrolyse du génome de C. thermocellum par les enzymes EcoRI, BamHI, HindIII et BglII, ceux des fragments de restriction obtenus qui pouvaient contenir les gènes codant pour les susdites enzymes cellulolytiques. La détection a été réalisée selon la méthode classique d'hybridation de Southern.

Les tailles des fragments de restriction obtenus figurent dans le tableau I ci-dessous. Ces fragments se sont révélés contenir celA et celB.

Tableau I

| Endonucléases | celA | celB |
|---|---|---|
| | kb | kb |
| BamHI | 6,2 9,7 | 14 |
| BglII | 26 | 4,4 11 |
| EcoRI | 9,7 | 6,0 |
| HindIII | 3,4 | 3,6 |

Des examens comparés de ces résultats et des analyses des restrictions effectuées tant sur ces fragments que sur les plasmides pCT101 et pCT203 ont été déduites les cartes de restriction des séquences correspondantes du génome. Ces cartes de restriction sont schématiquement indiquées dans la fig. 4, dans laquelle les lettres ont les significations suivantes :

B : BamHI, Bg : BglII, H : HindIII ; P : PstI, R : EcoRI. Les rectangles allongés apparaissant dans la fig. 4 correspondent aux fragments du génome qui contiennent respectivement les gènes celA et celB. Le fragment celA comprend moins de 3 kb, contient un site BamHI unique et est dépourvu de site BglII, EcoRI et HindIII. La séquence contenant celB contient moins de 3 kb, ne comporte qu'un seul site BglII et est dépourvu de site BamHI, EcoRI et HindIII.

Des fragments d'ADN contenant les susdites séquences et délimités par des sites HindIII ont été réinsérés dans des directions opposées dans pBR322. Ces fragments avaient des tailles de 3,4 et 3,6 kb respectivement. Il en est résulté deux paires de plasmides pour chacun des gènes celA et celB. Les plasmides pCT104 et pCT105 contiennent le fragment celA orienté dans deux directions opposées. Les plasmides pCT208 et pCT209 contiennent le fragment celB, également orienté dans deux directions opposées. Les parties concernées des plasmides ainsi obtenus, et à titre de comparaison, les parties correspondantes de pCT103 et pCT207 sont schématisées dans la fig. 5. Ces plasmides peuvent tous être utilisés pour transformer E. coli, par exemple E. coli HB101 ou des E. coli formant des « mini-cellules » (de l'anglais « minicells »), et les rendre aptes à produire d'importantes quantités d'enzyme cellulolytique, détectables et récupérables dans les conditions qui ont été indiquées plus haut. Le fait que les gènes contenus dans les susdits insérats soient exprimés, quelle que soit leur orientation dans les plasmides récepteurs, fonde la présomption que les insérats en question contiennent non seulement un gène susceptible d'être exprimé, mais les séquences qui, dans l'hôte naturel assurent la régulation de la transcription et de la traduction de ce gène. En outre, ces résultats soutiennent aussi l'hypothèse que les machineries contrôlant normalement la transcription et la traduction dans E. coli sont également capables de reconnaître ces éléments de régulation.

Essais immunologiques

Ils ont été réalisés avec un immunsérum préparé contre l'endo-β-1,4-glucanase de C. thermocellum purifiée selon la méthode décrite par Petre J. et Coll. (glucanase A).

L'immunsérum a été obtenu après injection à des lapins de 0,25 mg de l'enzyme purifiée préalablement émulsionnée dans l'adjuvant de Freund, en présence de sérum albumine bovine. Les injections ont été faites régulièrement toutes les trois semaines. Les sérums ont été prélevés sur ces lapins, après que ceux-ci eurent respectivement reçu trois injections d'enzyme.

Seul l'extrait d'E. coli 4282 (pCT1) donne, dans les tests d'immuno-diffusion d'Ouchterlony, une ligne de précipitation sans éperon avec celle formée par l'endo-β-1,4-glucanase de C. thermocellum testée dans des conditions analogues.

Des résultats semblables ont été obtenus avec les produits d'expression de E. coli 4282 (pCT104). Il est possible d'isoler après électrophorèse, notamment sur un gel de polyacrylamide, des polypeptides dans des bandes de poids moléculaires de 56 500, 55 000 et 50 200 (glucanases B). Elles sont immunoprécipitées par l'immunsérum susdit. Par contre, aucune réaction n'a été observée avec les produits d'expression des souches E. coli 4282 (pCT2) et E. coli 4282 (pHC 79).

De même, l'addition de 5μl de l'antisérum susdit à 50 μl d'extrait brut d'E. coli 4282 (pCT1) entraîne une inhibition de 85 % de l'activité CMCase mesurée par le test de viscosimétrie. Au contraire, aucune inhibition de ce type n'est observée avec l'extrait de E. coli 4282 (pCT 2).

De ces essais, il résulte que PCT1 porte vraisemblablement le gène de C. thermocellum codant pour l'endo-β1,4 glucanase connue de C. thermocellum. L'insérat contenu dans pCT2 contient un gène codant pour une autre enzyme cellulolytique également synthétisée par C. thermocellum. Cela paraît confirmé par le fait qu'un antisérum préalablement préparé contre la glucanase B contenue dans la bande de poids moléculaire de l'ordre de 66 000 (obtenue par séparation électrophorétique sur gel de polyacrylamide) parmi les produits d'expression de E. coli HB101 (pCT108) ne réagit pas avec les produits d'expression de E. coli 4182 (pCT1).

Les niveaux d'activité enzymatique produite par les deux souches de E. coli 4282 (pCT1) et E. coli 4282 (pCT2) et, plus généralement des souches de E. coli transformées par un recombinant contenant celA et celB, sont très élevées. En particulier, l'activité TNP-CMCase à 60 °C par mg de protéines de E. coli 4282 (pCT1) est de l'ordre de 30 % de celle qui peut être observée dans les mêmes conditions dans le milieu de culture de C. thermocellum, après 50 heures de croissance, c'est-à-dire à une vitesse de culture de l'ordre de quatre à cinq fois inférieure à celle de E. coli.

Les souches de E. coli transformées par l'un ou/et l'autre des recombinants sus-décrits sont des micro-organismes de choix pour produire dans les conditions qui ont été définies plus haut une activité cellulolytique en raison des conditions de culture industriellement favorables telles que :

rapidité de multiplication,

culture en anaérobiose non stricte,

utilisation de substrats bon marché tels que mélasses.

L'invention concerne donc encore en particulier des micro-organismes aérobies stricts ou facultatifs, caractérisés par leur capacité à produire une enzyme cellulolytique reconnue par un immunsérum, préalablement formé contre au moins une enzyme cellulolytique produite par C. thermocellum. En particulier, l'invention concerne un micro-organisme aérobie strict ou facultatif, produisant au moins une enzyme cellulolytique ayant un poids moléculaire de l'ordre de 50 200, 55 000, 56 500 ou 66 000. A titre de micro-organisme préféré, on citera ceux qui produisent une enzyme cellulolytique reconnue par un immunsérum, préalablement formé contre l'une des glucanases A ou B codées par tout ou partie du gène celA ou celB. Il va de soi que l'on peut avoir recours pour cette reconnaissance à des réactions immunologiques appropriées in vitro, notamment par immuno-précipitation. Les micro-organismes préférés sont dérivés de E. coli. Ils sont capables notamment de fournir des extraits bruts présentant des activités CMCase et/ou TNP-CMCase élevées.

Les indications de poids moléculaires qui précèdent ont été appréciées par rapport aux distances de migration de protéines étalons dans un même système électrophorétique, sur gel de polyacrylamide-dodécyl-sulfate de sodium à 10 %, selon la technique de Lämmli, U.K. (1970). « Cleavage of structural proteins during the assembly of the head of bacteriophage T4 ». Nature 227 : 680-685 : myosine 200 kdal ; phosphorylase b 92,5 kdal ; sérumalbumine bovine 69 kdal ; ovalbumine 46 kdal ; anhydrase carbonique 30 kdal ; lyzozyme 14,3 kdal.

Les séparations de fragments d'ADN dont question plus haut ont été effectuées dans un tampon borate comme décrit par Maniatis et Coll. (« Molecular cloning, a laboratory manual ». Cold Spring Harbor Laboratory, New York). Les fragments de restriction ont été récupérés à partir des gels par électro-élution. D'une façon générale, la transformation de E. coli a été effectuée selon la méthode de Cohen et Coll. (« Non chromosomal antibiotic resistance in bacteria : genetic transformation of Escherichia coli by R-factor DNA ». Proc. Natl. Acad. Sci. 69 : 2110-2114). Les sondes d'ADN ont été marquées avec ($\alpha$-$^{32}$P) d'ATP par la technique dite de « nick-translation » (Rigby et Coll. 1977. « Labeling deoxyribonucleic acid to high specific activity in vitro by nick translation with DNA polymerase ». I. J. Mol. Biol. 113 : 237-25). Les hybridations ont été réalisées par la méthode de Southern (1975. « Detection of specific sequences among DNA fragments separated by gel electrophoresis ». J. Mol. Biol. 98 : 503-517).

L'invention ne se limite évidemment pas aux modes de réalisation décrits ci-dessus à titre d'exemples et l'homme de métier peut y apporter des modifications, sans pour autant sortir du cadre des revendications ci-après ; bien entendu, on peut avoir recours à tous mutants ou variants de E. coli ou à de nombreux micro-organismes autres que E. coli, dans la mesure où ils ont été rendus capables de synthétiser les susdites enzymes cellulolytiques. Il s'agit par exemple d'autres entérobactéries, ou d'autres genres ou espèces, telles que les bacillaceae, par exemple B. subtilis, ou encore de levures, par exemple Saccharomyces cerevisiae. Il est entendu que l'on aura recours dans chaque cas aux vecteurs appropriés, susceptibles de transformer le micro-organisme choisi et dont de nombreux exemples ont déjà été décrits dans la littérature, lesdits vecteurs ayant naturellement au préalable été modifiés par une séquence originaire de C. thermocellum et apte à coder pour une enzyme douée de propriétés cellulolytiques. Dans le cas de S. cerevisiae, il s'agit par exemple de levures transformées par le plasmide 2μ, dans lequel aura été insérée l'une des séquences celA ou celB décrites ci-dessus.

D'une façon générale, on aura recours à un vecteur ainsi modifié, dans lequel la séquence originaire de C. thermocellum aura, si besoin, été insérée à proximité d'un promoteur ou d'une séquence susceptible d'agir en tant que promoteur, ledit promoteur étant apte à être reconnu par une ARN-polymérase endocellulaire du micro-organisme destiné à être transformé par ledit vecteur.

**Revendications**

1. Micro-organisme aérobie strict ou facultatif, transformé un vecteur contenant un insérat issu du patrimoine génétique de C. thermocellum et contenant une séquence d'ADN codant pour une enzyme cellulolytique, cet insérat étant placé dans ledit vecteur, dans des conditions permettant l'expression dans ce micro-organisme distinct de C. thermocellum de la séquence codant pour l'enzyme cellulolytique.

2. Micro-organisme selon la revendication 1, caractérisé en ce qu'il est dérivé de E. coli.

3. Micro-organisme selon la revendication 1 ou la revendication 2, caractérisé en ce qu'il est transformé par un cosmide contenant la susdite séquence d'ADN de C. thermocellum, ce cosmide étant du type de ceux qui permettent l'empaquetage relativement sélectif de fragments comportant de 25 à 50 kilobases.

4. Micro-organisme selon la revendication 1 ou la revendication 2, caractérisé en ce que le susdit vecteur consiste en un plasmide ou phage contenant la susdite séquence d'ADN.

5. Micro-organisme selon l'une quelconque des revendications 1 à 4, caractérisé en ce que le susdit insérat contient aussi les éléments qui assurent la régulation de la transcription et de la traduction de la séquence codant pour l'enzyme cellulolytique dans C. thermocellum.

6. Vecteur pour la transformation d'un micro-organisme aérobie strict ou facultatif, et contenant un promoteur reconnu par une ARN-polymérase endocellulaire dudit micro-organisme, caractérisé par la présence d'un insérat contenant une séquence d'ADN issue de C. thermocellum, codant pour une enzyme cellulolytique, et placée sous le contrôle dudit promoteur dans des conditions permettant son expression, lorsque ledit vecteur a été incorporé au micro-organisme susdit.

7. Vecteur selon la revendication 6, caractérisé en ce qu'il est apte à transformer E. coli.

8. Vecteur selon la revendication 6 ou la revendication 7, caractérisé en ce que ledit promoteur correspond à celui qui est normalement associé à ladite séquence d'ADN dans C. thermocellum.

9. Composition présentant une activité cellulolytique, caractérisée en ce qu'elle consiste en une masse de micro-organismes transformés selon l'une quelconque des revendications 1 à 5, essentiellement dépourvus de parois cellulaires, cette composition étant encore caractérisée par la capacité des extraits desdits micro-organismes d'hydrolyser la trinitrophényl-carboxyméthyl-cellulose et par la présence dans cette dernière d'enzymes reconnues par des anticorps reconnaissant, soit l'endo-bêta-1,4-glucanase extracellulaire de C. thermocellum, soit une glucanase A, soit encore une glucanase B de C. thermocellum.

10. Composition selon la revendication 9, caractérisée en ce que, lorsque les susdits micro-organismes sont utilisés à raison d'une quantité correspondant à 65 microgrammes de protéines, ils présentent une activité TNP-CMCase d'au moins 0,05 unités d'absorbance d'une radiation de 350 nm par heure, après incubation du mélange à 60 °C, étant entendu que les mesures de variations d'absorbance s'expriment par rapport à l'action du mélange sur le substrat chromogène constitué par la TNP-CMC, préparée selon la méthode de Huang et Tang (Anal. Biochem. (1976), 73, 369-377) et par rapport à l'absorption mesurée sur un mélange des constituants évoqués plus haut, après incubation dans les mêmes conditions, cependant en l'absence de l'extrait cellulaire.

11. Composition selon la revendication 9 ou la revendication 10, caractérisée en ce qu'elle consiste en une masse de E. coli transformé.

12. Composition selon la revendication 11, caractérisée en ce qu'elle est constituée d'un extrait brut de E. coli transformé, conduisant, lorsqu'il est mis en contact avec une solution de 1,5 % (poids/volume) de carboxyméthyl-cellulose dans un tampon PC (50 mM $K_2HPO_4$ ; acide citrique 12,5 mM ; pH 6,3) à raison d'une quantité d'extrait brut contenant 65 microgrammes de protéine, diluée dans 50 microlitres du même tampon, par 1 ml de la susdite solution, à une réduction de moitié de la viscosité initiale après 30 minutes et de préférence 10 minutes d'incubation du mélange formé à 60 °C.

13. Composition selon la revendication 11, caractérisée en ce qu'elle est constituée d'un extrait brut de E. coli transformé, conduisant, lorsqu'il est mis en contact avec une solution 1,5 % (poids/volume) de carboxyméthyl-cellulose dans un tampon PC (50 mM $K_2HPO_4$ ; acide citrique 12,5 mM ; pH 6,3) à raison d'une quantité d'extrait brut contenant 65 microgrammes de protéine, diluée dans 50 microlitres du même tampon, par 1 ml de la susdite solution, à une activité CMCase exprimée en % d'équivalents de glucose libéré d'au moins 0,3 %, de préférence au moins 1 %, après incubation du mélange à 60 °C pendant 1 heure.

14. Procédé pour la dégradation cellulolytique de matières cellulosiques comprenant la mise en contact de ces matières cellulosiques avec la composition selon l'une quelconque des revendications 9 à 13, à une température de l'ordre de 50 à 70 °C, de préférence de l'ordre de 60° et par l'incubation du mélange à cette température pendant le temps nécessaire à la réalisation de la lyse contrôlée desdites matières cellulosiques, plus particulièrement de leurs liaisons bêta-1,4-glycosyle.

**Claims**

1. Microorganism, strictly or optionally aerobic, transformed by a vector containing an insert derived from the genetic patrimony of C. thermocellum and containing a DNA sequence coding for a cellulolytic enzyme, said insert being placed in said vector under conditions which allow the expression of the sequence coding for the cellulolytic enzyme in the microorganism, said microorganism being different from C. thermocellum.

2. Microorganism according to claim 1, characterized in that it is derived from E. coli.

3. Microorganism according to claim 1 or claim 2, characterized in that it is transformed by a cosmid containing said DNA sequence of C. thermocellum, said cosmid being of the type which allow the relatively selective packaging of fragments comprising 25 to 50 kilo-bases.

4. Microorganism according to claim 1 or claim 2, characterized in that said vector consists of a plasmid or a phage containing said DNA sequence.

5. Microorganism according to any of claims 1 to 4, characterized in that said insert also contains the regulatory elements which control the transcription and the translation of the sequence coding for the cellulolytic enzyme in C. thermocellum.

6. Vector for the transformation of a strictly or optionally aerobic microorganism, said vector containing a promoter recognizable by an endocellular RNA-polymerase of said microorganism, characterized by the presence of an insert containing a DNA sequence derived from C. thermocellum, coding for a cellulolytic enzyme and placed under the control of said promoter under conditions which allow its expression when said vector is incorporated into said microorganism.

7. Vector according to claim 6, characterized in that it is capable of transforming E. coli.

8. Vector according to claim 6 or claim 7, characterized in that said promoter corresponds to that which is normally associated with said DNA sequence in C. thermocellum.

9. Composition having a cellulolytic activity, characterized in that it comprises a mass of transformed microorganisms according to any of claims 1 to 5, essentially free of cell walls, said composition being further characterized by the ability of extracts of said microorganisms to hydrolyse trinitro-phenyl-carboxymethyl-cellulose and by the presence in the latter of enzymes recognised by antibodies recognising either extracellular endo-β-1,4-glucanase of C. thermocellum or a glucanase A or a glucanase B of C. thermocellum.

10. Composition according to claim 9, characterized in that when said microorganisms are used in an amount corresponding to 65 micrograms of protein, they possess a TNP-CMCase activity of at least 0.05 absorbance units of radiation of 350 nm per hour, after incubation of the mixture at 60 °C, the measurements of absorbance variation being expressed with respect to the action of the mixture on the chromogen substrate consisting of TNP-CMC, prepared by the method of Huang and Tang (Anal. Biochem. (1976), 73, 369-377) and with respect to the absorption measured on a mixture of the constituants mentioned above, after incubation under the same conditions, but in the absence of cellular extract.

11. Composition according to claim 9 or claim 10, characterized in that it comprises a mass of transformed E. coli.

12. Composition according to claim 11 characterized in that it comprises a crude extract of transformed E. coli, leading to, when H is contacted with a 1.5 % (weight/volume) solution of carboxymethylcellulose in a PC buffer (50 mM $K_2HPO_4$ ; citric acid 12.5 mM ; pH 6.3) in an amount of crude extract containing 65 micrograms of protein, diluted in 50 microlitres of the same buffer by 1 ml of the above said solution, to a reduction of half the initial viscosity after 30 minutes and preferably 10 minutes of incubation of the mixture formed at 60 °C.

13. Composition according to claim 11 characterized in that it comprises a crude extract of transformed E. coli, leading to, when it is contacted with a 1.5 % (weight/volume) solution of carboxymethyl cellulose in a P.C. buffer (50 mM $K_2HPO_4$ ; citric acid 12.5 mM ; pH 6.3) in an amount of crude extract containing 65 micrograms of protein, diluted in 50 microlitres of the same buffer, by 1 ml of

the abovesaid solution, to a CMCase activity expressed in percent equivalents of glucose liberated of at least 0,3 % preferably at least 1 % after incubation of the mixture at 60 % for 1 hour.

14. Process for the cellulolytic degradation of cellulosic materials comprising the contacting of the cellulosic materials with the composition according to any of claims 9 to 13 at a temperature of the order of 50 to 70 °C, preferably of the order of 60 °C, and the incubation of the mixture at this temperature for the time necessary for the realisation of the controlled lysis of the said cellulosic materials, more particularly of their β-1,4-glycosyl bonds.

## Patentansprüche

1. Obligat oder fakultativ aerober Mikroorganismus, der durch einen Vektor transformiert ist, welcher ein aus der Erbmasse von C. thermocellum stammendes Insert, das eine DNS-Sequenz, die ein zellulolytisches Enzym kodiert, enthält, wobei das Insert in besagtem Vektor inseriert wurde, unter Bedingungen, welche die Expression, der das zellulolytische Enzym kodierenden Sequenz in diesem von C. thermocellum verschiedenen Mikroorganismus erlauben.

2. Mikroorganismus nach Anspruch 1, dadurch gekennzeichnet, daß er von E. coli abgeleitet ist.

3. Mikroorganismus nach Anspruch 1 oder 2, dadurch gekennzeichnet, daß er durch ein Kosmid transformiert ist, welches Kosmid die besagte DNS-Sequenz von C. thermocellum enthält und einen Typ darstellt, der die relativ selektive Umhüllung von Fragmente, die 25 bis 50 kilobasen umfassen, erlaubt.

4. Mikroorganismus nach Anspruch 1 oder 2, dadurch gekennzeichnet, daß der besagte Vektor aus einem Plasmid oder Phagen besteht, das bzw. der besagte DNS-Sequenz enthält.

5. Mikroorganismus nach einem der Ansprüche 1 bis 4, dadurch gekennzeichnet, daß das besagte Insert auch jene Elemente enthält, die die Steuerung der Transkription und der Traduktion der Sequenz, die das zellulolytische Enzym kodiert in C. thermocellum gewährleistet.

6. Vektor zur Transformation eines obligat oder fakultativ aeroben Mikroorganismus, der einen, durch eine endozelluläre RNS-Polymerase des besagten Mikroorganismus erkannten Promotor enthält, gekennzeichnet durch die Gegenwart eines Inserts, das eine aus C. thermocellum stammende DNS-Sequenz, die ein zellulolytisches Enzym kodiert, enthält und der Kontrolle des besagten Promotors unter Bedingungen unterliegt, die seine Expression erlauben, wenn der Vektor in den oben gennanten Mikroorganismus eingebaut worden ist.

7. Vektor nach Anspruch 6, dadurch gekennzeichnet, daß er imstande ist, E. coli zu transformieren.

8. Vektor nach Anspruch 6 oder 7, dadurch gekennzeichnet, daß der besagte Promotor demjenigen entspricht, der normalerweise mit der besagten DNS-Sequenz in C. thermocellum verbunden ist.

9. Zusammensetzung, die eine zellulolytische Aktivität aufweist, dadurch gekennzeichnet, daß sie aus einer Masse der nach den Ansprüchen 1 bis 5 transformierten Mikroorganismen besteht, im wesentlichen ohne Zellwände, wobei diese Zusammensetzung noch durch die Fähigkeit der Extrakte der besagten Mikroorganismen, die Trinitrophenyl-Carboxymethyl-Zellulose zu hydrolisieren, gekennzeichnet ist, sowie durch die Gegenwart in letzterer von Enzymen, die durch Antikörper, die entweder extrazelluläre Endo-beta-1,4-glucanase von C. thermocellum oder eine Glucanase A oder außerdem noch Glucanase B von C. thermocellum erkennen, erkannt wurden.

10. Zusammensetzung nach Anspruch 9, dadurch gekennzeichnet, daß, wenn die oben genannten Mikroorganismen in einer Menge verwendet werden, die 65 Mikrogramm Proteinen entspricht, sie eine TNP-CMCase-Aktivität von wenigstens 0.05 Absorptionseinheiten einer Strahlung von 350 nm pro Stunde aufweisen, nach Inkubation der Mischung bei 60 °C, vorausgesetzt, daß die Messungen der Absorptionsvariationen im Verhältnis zur Wirkung der Mischung auf das von der TNP-CMC gebildete chromogene Substrat, hergestellt nach der Methode von Huang und Tang (Anal. Bioch. (1976), 73, 369-377), und im Verhältnis zu der an einer Mischung der oben erwähnten Komponenten gemessenen Absorption, nach Inkubation unter denselben Bedingungen, jedoch in Abwesenheit von Zellextrakt ausgedrückt werden.

11. Zusammensetzung nach Anspruch 9 oder 10, dadurch gekennzeichnet, daß sie aus einer Masse von transformiertem E. Coli besteht.

12. Zusammensetzung nach Anspruch 11, dadurch gekennzeichnet, daß sie aus einem Rohextrakt von transformiertem E. coli gebildet ist, der, wenn er mit einer Lösung von 1,5 % (Gewicht/Volumen) Carboxymethyl-Zellulose in einem PC-Puffer (50 mM $K_2HPO_4$ ; Zitronensäure 12,5 mM ; pH 6,3) in Kontakt gebracht wird, wobei die Menge Rohextrakt 65 Mikrogramm Protein enthält, in 50 Mikroliter desselben Puffers durch 1 ml der genannten Lösung verdünnt ist, nach 30 Minuten, und vorzugsweise nach 10 Minuten Inkubation der bei 60 °C gebildeten Mischung zu einer Reduktion der Anfangsviskosität um die Hälfte führt.

13. Zusammensetzung nach Anspruch 11, dadurch gekennzeichnet, daß sie aus einem Rohextrakt von transformierten E. coli gebildet ist, der, wenn er mit einer Lösung von 1,5 % (Gewicht/Volumen) Carboxymethyl-Zellulose in einem PC-Puffer (50 mM $K_2HPO_4$ ; Zitronensäure 12,5 mM ; pH 6,3) in Kontakt gebracht wird, wobei die Menge Rohextrakt 65 Mikrogramm Protein enthält, in 50 Mikroliter desselben Puffers durch 1 ml der genannten Lösung verdünnt ist, zu einer in Äquivalentprozende der freigesetzten Glucose ausgedrückten CMCase-Aktivität von wenigstens 0,3 %, vorzugsweise von wenigstens 1 % nach Inkubation der Mischung bei 60 °C während 1 Stunde, führt.

14. Verfahren zum zellulolytischen Abbau von Zellulosestoffen, welche die Kontaktierung dieser Zellulosestoffe mit der Zusammensetzung nach einem der Ansprüche 9 bis 13 umfaßt, bei einer Temperatur in der Größenordnung von 50 bis 70 °C, vorzugsweise in der Größenordnung von 60 °C, und durch Inkubation der Mischung bei dieser Temperatur während einer Zeit, die notwendig ist, um die kontrollierte Auflösung der besagten Zellulosestoffe, insbesondere ihrer β-1,4-Glycosylbindungen zu realisieren.

Fig.1.

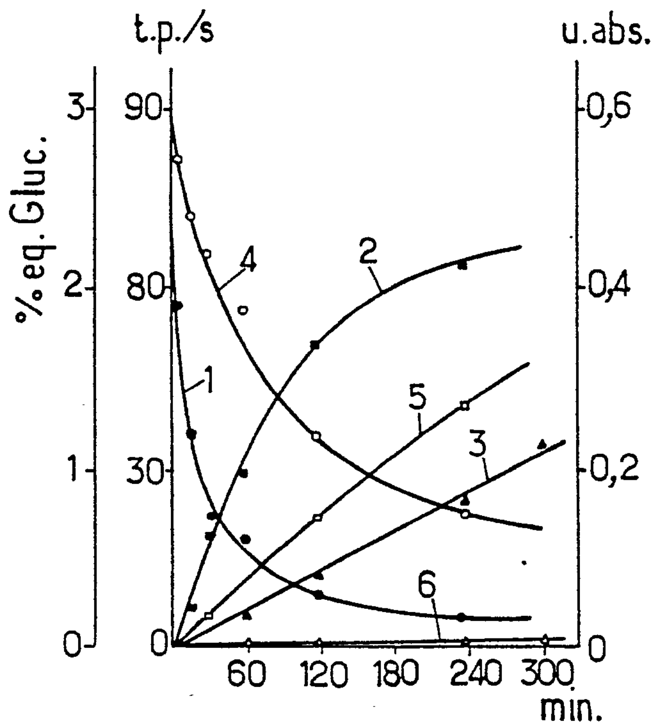
t.p./s
u.abs.
% eq. Gluc.
3
2
1
0
90
80
30
0
0,6
0,4
0,2
0
1
2
3
4
5
6
60
120
180
240
300
min.

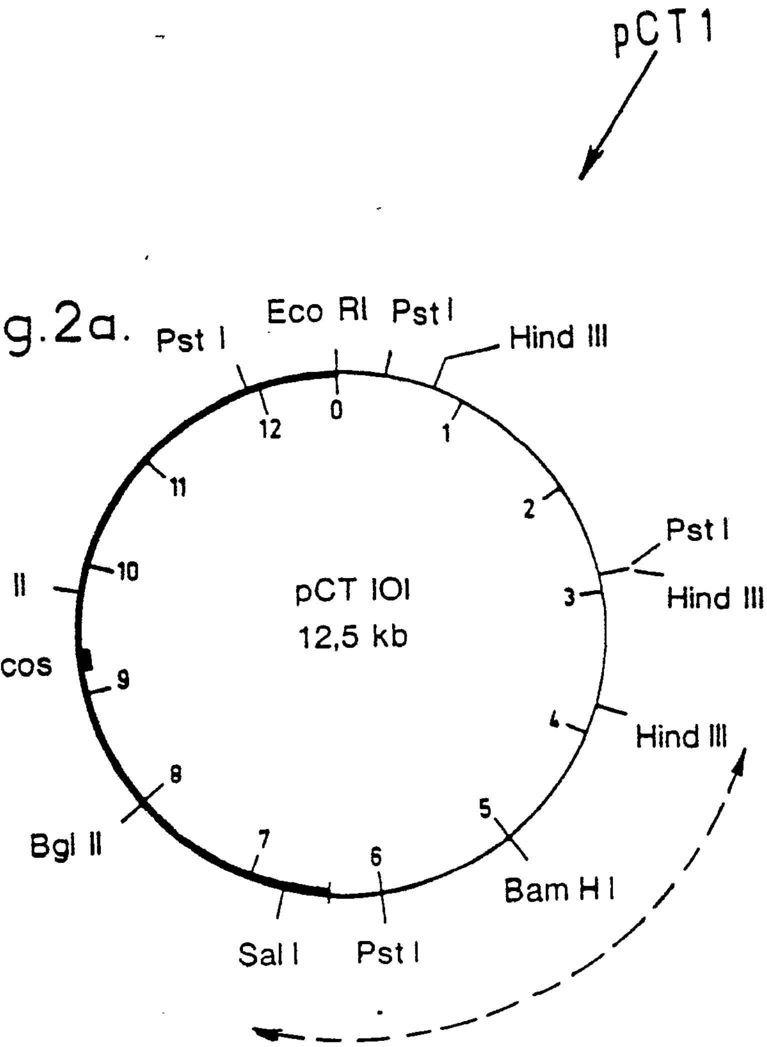
pCT1
Eco RI
Pst I
Pst I
Hind III
Pst I
Hind III
Hind III
Bam H I
Pst I
Sal I
Bgl II
cos
Bgl II
pCT 101
12,5 kb
0
1
2
3
4
5
6
7
8
9
10
11
12

Fig.2a.

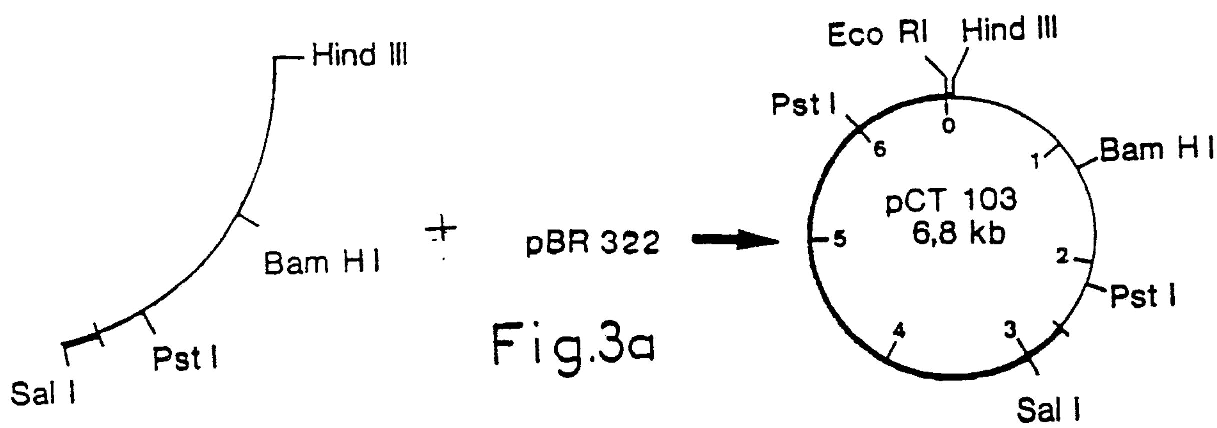
Hind III
Bam H I
Pst I
Sal I
+
pBR 322
Eco RI
Hind III
Pst I
Bam H I
pCT 103
6,8 kb
Pst I
Sal I
0
1
2
3
4
5
6

Fig.3a

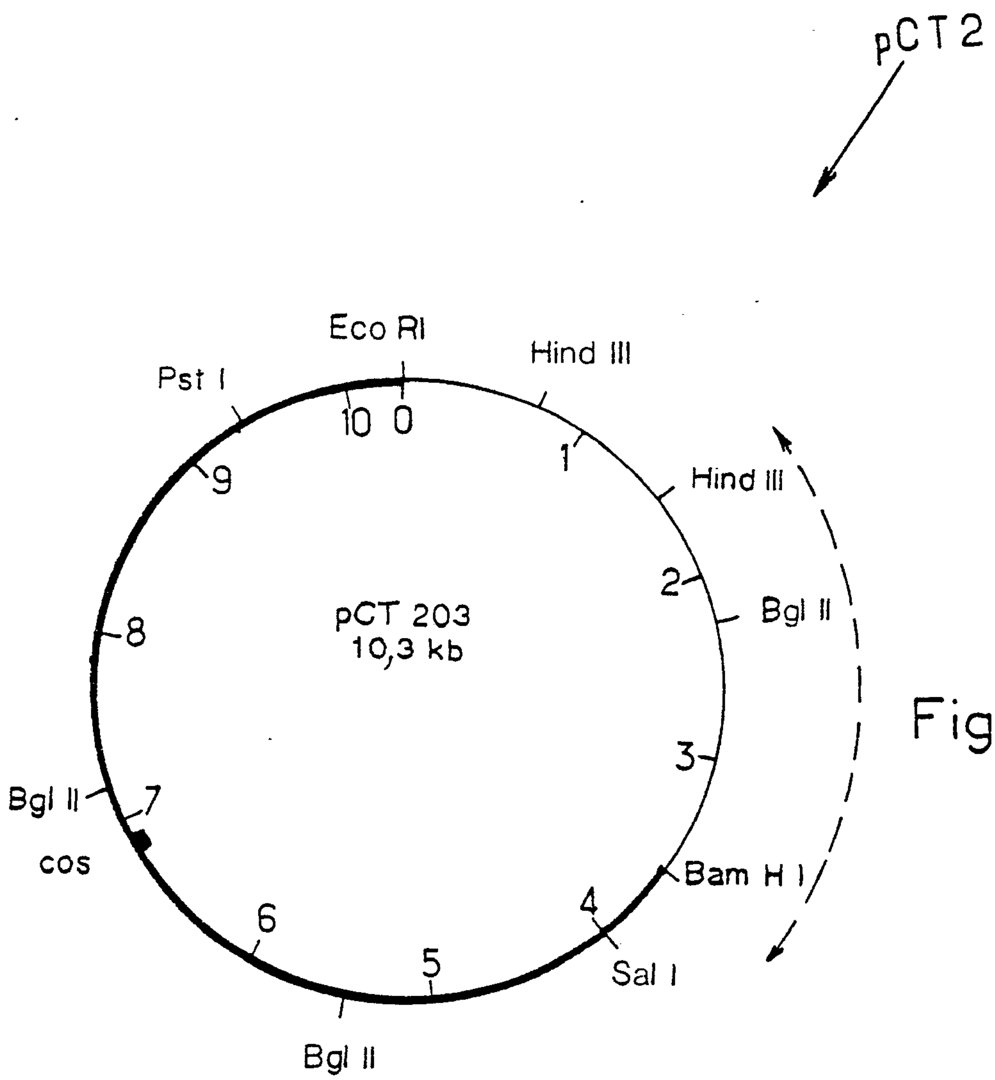
pCT2
Eco RI
Hind III
Pst I
10 0
1
Hind III
9
2
Bgl II
pCT 203
10,3 kb
8
3
Bgl II
7
cos
Bam H I
4
6
5
Sal I
Bgl II

Fig.2b.

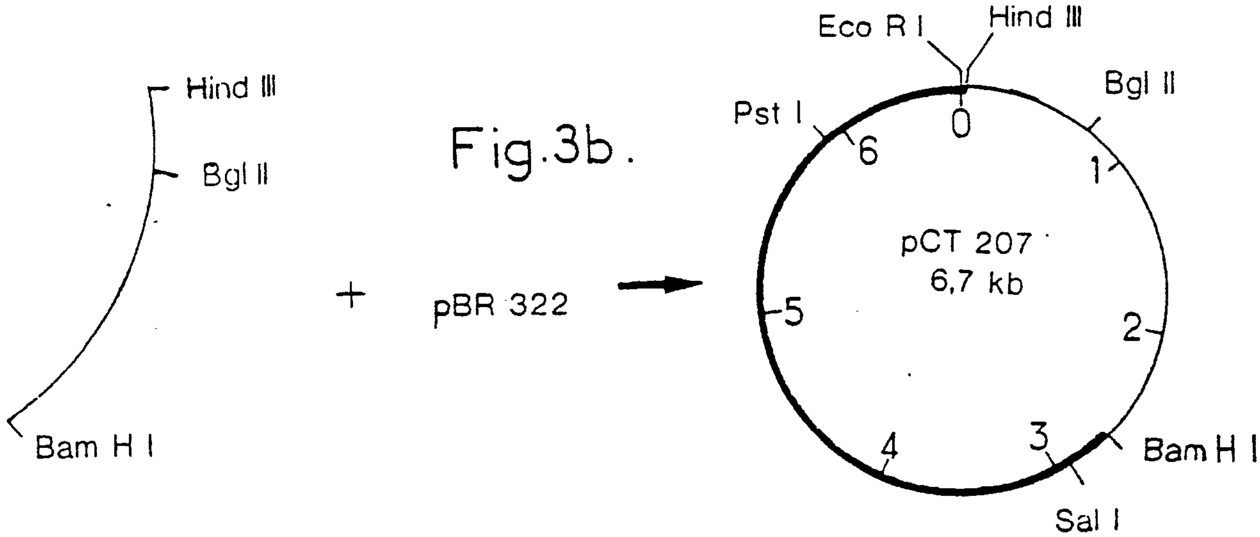
Hind III
Bgl II
Bam H I
+
pBR 322
Eco R I
Hind III
Pst I
6
0
Bgl II
1
pCT 207
6,7 kb
5
2
4
3
Bam H I
Sal I

Fig.3b.

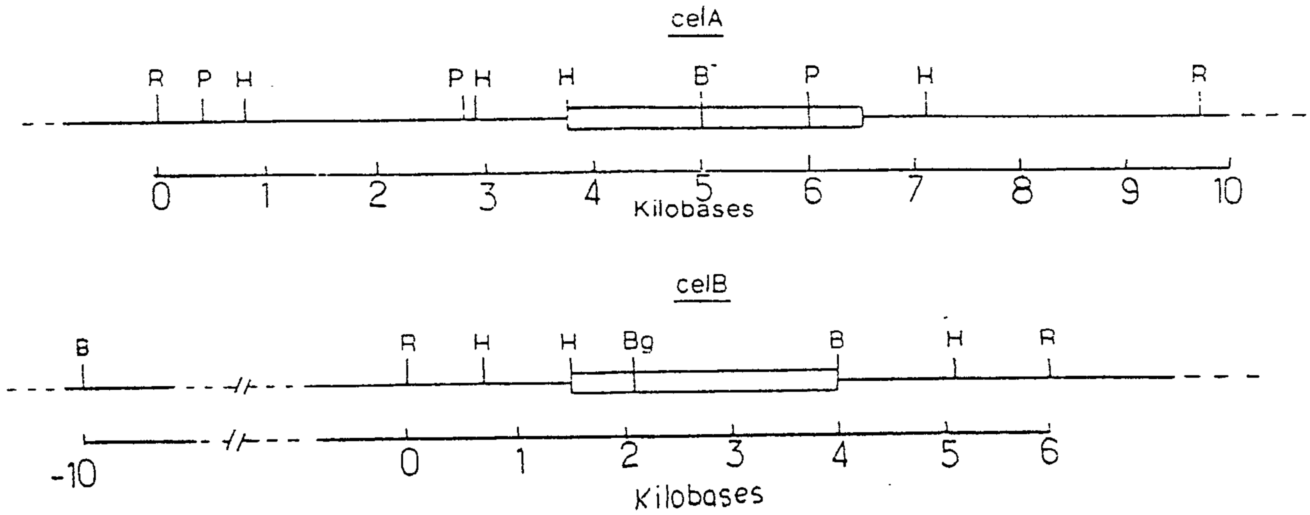
celA
R
P
H
P
H
H
B
P
H
R
0
1
2
3
4
5
6
7
8
9
10
Kilobases
celB
B
R
H
H
Bg
B
H
R
-10
0
1
2
3
4
5
6
Kilobases

Fig.4.

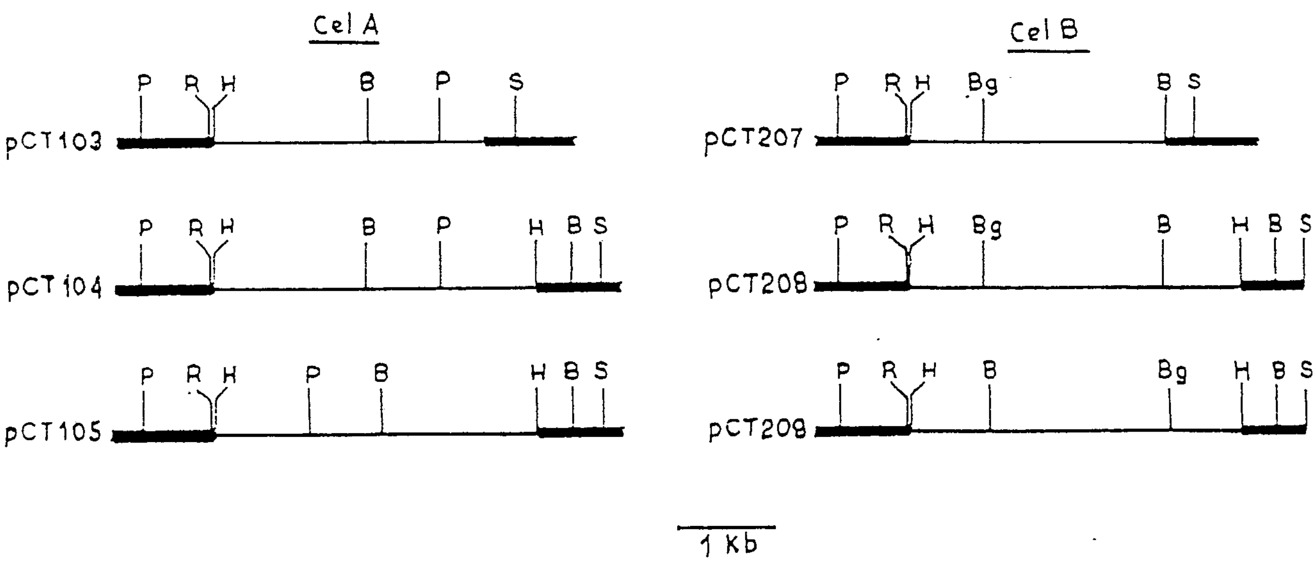
Cel A
Cel B
pCT103
pCT104
pCT105
pCT207
pCT208
pCT209
P
R
H
B
S
Bg
1 Kb

Fig.5.